# EUROPEAN PATENT SPECIFICATION

(11) **EP 4 013 863 B1**
(45) Date of publication and mention of the grant of the patent: **29.07.2026**
(21) Application number: 20851793.8
(22) Date of filing: 14.08.2020
(51) Int. Cl.: C12N 15/09, C12N 15/10, C12N 15/13, C12P 19/34, C12Q 1/6869, C12Q 1/6806, C12Q 1/6834

(54) **PROBE-CAPTURE METHOD FOR TCR ALPHA AND BETA CHAIN VDJ-RECOVERY FROM OLIGO-DT REVERSE TRANSCRIBED RNA**
SONDENERFASSUNGSVERFAHREN FÜR TCR-ALPHA- UND BETA-KETTEN-VDJ-RÜCKGEWINNUNG AUS EINER OLIGO-DT-RÜCKTRANSKRIBIERTEN RNA
PROCÉDÉ DE CAPTURE DE SONDE POUR LA RÉCUPÉRATION DE VDJ DE CHAÎNE BÊTA DE TCR ALPHA ET BÊTA À PARTIR D'ARN TRANSCRIT INVERSE D'OLIGO-DT

(30) Priority: 14.08.2019 US 201962886663 P
(43) Date of publication of application: 22.06.2022
(73) Proprietor: iRepertoire, Inc., Huntsville AL 35806 (US)
(72) Inventor: PAN, Wenjing, Huntsville, AL 35806 (US); BYRNE-STEEL, Miranda, Huntsville, AL 35806 (US); BROWN, Brittany, Huntsville, AL 35806 (US); SONG, Li, Huntsville, AL 35806 (US); HAN, Jian, Huntsville, AL 35806 (US)
(74) Representative: Dehns
(86) International application number: PCT/US2020/046433
(87) International publication number: WO 2021/030716

(56) References cited:
- WO-A1-2018/013723
- WO-A1-2018/075693
- US-A1- 2015 329 891
- US-A1- 2015 329 891
- US-A1- 2018 208 984
- US-B2- 9 512 487
- W. MIACHEL HANSON ET AL: "Reversible Oligonucleotide Chain Blocking Enables Bead Capture and Amplification of T-Cell Receptor α and &bgr; Chain mRNAs", JOURNAL OF THE AMERICAN CHEMICAL SOCIETY, vol. 138, no. 35, 7 September 2016 (2016-09-07), pages 11073 - 11076, XP055336990, ISSN: 0002-7863, DOI: 10.1021/jacs.6b04465

## Description

### Cross-Reference to Related Application

This application claims priority to U.S. Provisional Patent Application No. 62/886,663, entitled "Probe-Capture Method for TCR Alpha and Beta Chain VDF-Recovery from Oligo-DT Reverse Transcribed RNA" and filed on August 14, 2019.

### Background of the Invention

Single cell transcriptional analysis reveals a wealth of information that is obscured when analyzing the RNA of a sample *en masse.* Furthermore, it is important to know the identity of the T Cell Receptor (TCR) or B Cell Receptor (BCR) chain VDJ-rearrangements associated with the phenotypic state of each individual cell. A TCR gene consists of numerous V regions (variable region, V), J regions (joining region, J), D regions (diversity region, D), and C regions (constant region, C) encoded by different regions in the genome. In the T cell differentiation process, such gene fragments are genetically rearranged in various combinations. This information is valuable as it can provide direct calculations of clonal frequency in various cell subsets, tracking of specific lymphocytes with treatment, and reveal paired information for both chains of the receptor for downstream functional analysis or Car-T development. Many single cell systems on the market utilize an oligo-dT-based reverse transcription (RT) step in which the RNA from an isolated single cell is reverse transcribed using a barcoded oligo-dT primer. The resulting RNA sequencing can provide information related to various genes expressed in the cell traced back to the same cell via the barcode introduced during RT, but this method cannot reliably reveal the sequence of the paired-receptor VDJ-rearrangement.

This deficiency is due, in part, to the fact that the *de novo* VDJ-rearrangement is far from the poly-A tail of the RNA making sequencing on certain high throughput next-generation sequencing platforms a challenge, because the resulting products are 1-1.5 kb in length. This can be overcome by switching to longer read length NGS platforms or using commercially available Illumina sequencing kits in a unique manner. This switch, however, still does not overcome the deficiencies of conventional methods.

US 2015/329891 relates to methods and compositions for analyzing nucleic acids associated with single cells using nucleic acid barcodes. Hanson et al. (Journal of the American Chemical Society (2016) vol. 138, no. 35, pages 11073 - 11076) discloses that reversible oligonucleotide chain blocking enables bead capture and amplification of T-cell receptor alpha and beta chain mRNAs. US 9 512 487 relates to methods for using DNA sequencing to identify personalized, or patient-specific biomarkers in patients with lymphoid neoplasms, autoimmune disease and other conditions. US 2018/208984 relates to methods, compositions, kits, and systems useful in the determination and evaluation of the immune repertoire. WO 2018/075693 relates to methods, compositions and systems for analyzing individual cells or cell populations through a partitioned analysis of contents of individual cells or cell populations, such as cancer cells and cells of the immune system.

### Summary of the Invention

The present invention relates to a method comprising the steps of: reverse transcribing at least one first strand of cDNA from mRNA using an oligo-dT primer, wherein the mRNA comprises at least one target sequence to produce a first strand cDNA and wherein the oligo-dT primer comprises an engineered sequence on the 5' end; producing a first set of amplicons by amplifying the first strand cDNA using a multiplex primer mix, wherein the multiplex primer mix comprises two or more primers configured to bind two or more sequences selected from the group consisting of: TCR alpha chain VDJ and TCR beta chain VDJ sequences, and a reverse primer configured to bind to the engineered sequence on the 5' end of the oligo-dT primer wherein the two or more primers of the multiplex primer mix are an asymmetric balance of primers configured to favor amplification of the sense strand VDJ sequences; using probe beads to capture amplicons comprising TCR alpha chain VDJ sequences and TCR beta chain VDJ sequences from the first set of amplicons, wherein the probe beads comprise targeted probe(s) in J or C genes of the TCR alpha chain and the TCR beta chain; washing the sample to remove uncaptured amplicons from the first set of amplicons; eluting captured amplicons from the probe beads to produce a pool of eluted amplicons; and amplifying the eluted amplicons using PCR to produce a second set of amplicons.

In some embodiments of the invention, the method further comprises the step of, after amplifying the eluted amplicons, sequencing the second set of amplicons using next generation sequencing. In some embodiments of the invention, the method further comprises the step of, after sequencing the second set of amplicons using next generation sequencing, evaluating the sequences to determine the frequency of TCR alpha chain VDJ sequences and TCR beta chain VDJ sequences.

In some embodiments of the invention, the probe bead primers used in the method are configured to bind to at least one location within the TCR alpha chain constant gene region and at least one location within the TCR beta chain constant gene region. In some embodiments of the invention, the probe bead primers used in the method are configured to bind to at least one location within the TCR alpha chain constant gene region. In some embodiments of the invention, the probe bead primers used in the method are configured to bind to at least one location within the TCR beta chain constant gene region.

In some embodiments of the invention, the oligo-dT primer used in the method comprises a molecular barcode. In some embodiments of the invention, the multiplex primer mix used in the method comprises an engineered sequence on the 5' end of the primers and which is configured as a universal binding site.

In some embodiments of the invention, prior to using probe beads, the first set of amplicons is cleaned by SPRI bead selection and an additional round of PCR is performed.

### Brief Description of the Drawings

The disclosure can be better understood with reference to the following drawings. The elements of the drawings are not necessarily to scale relative to each other, emphasis instead being placed upon clearly illustrating the principles of the disclosure. Furthermore, like reference numerals designate corresponding parts throughout the several views.

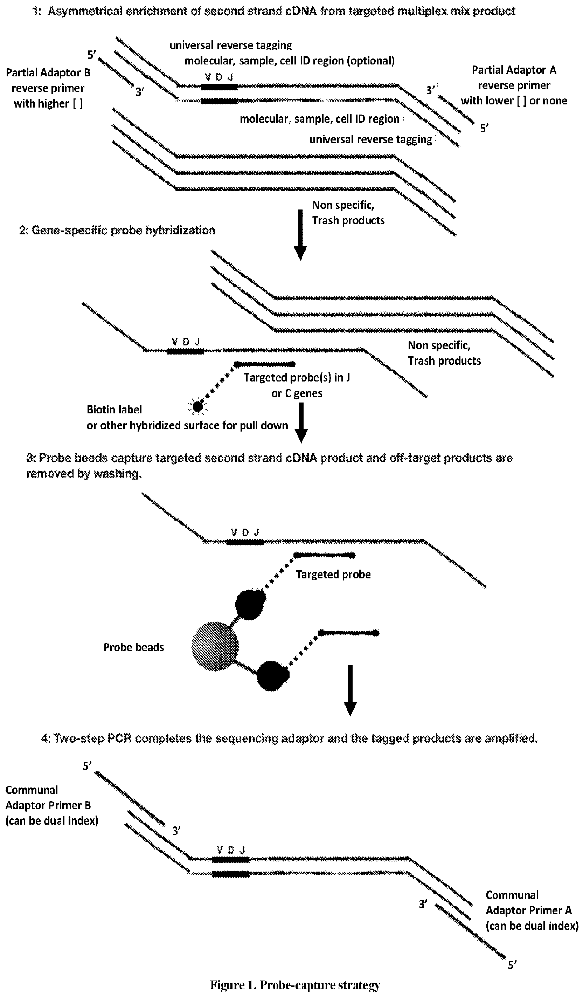

FIG. 1 displays four stages of a probe-capture strategy including asymmetrical enrichment of second strand cDNA from targeted multiplex mix product, gene-specific probe hybridization, capture of targeted second-strand cDNA product and removal of off-target products by washing, and two-step PCR and amplification of tagged products.

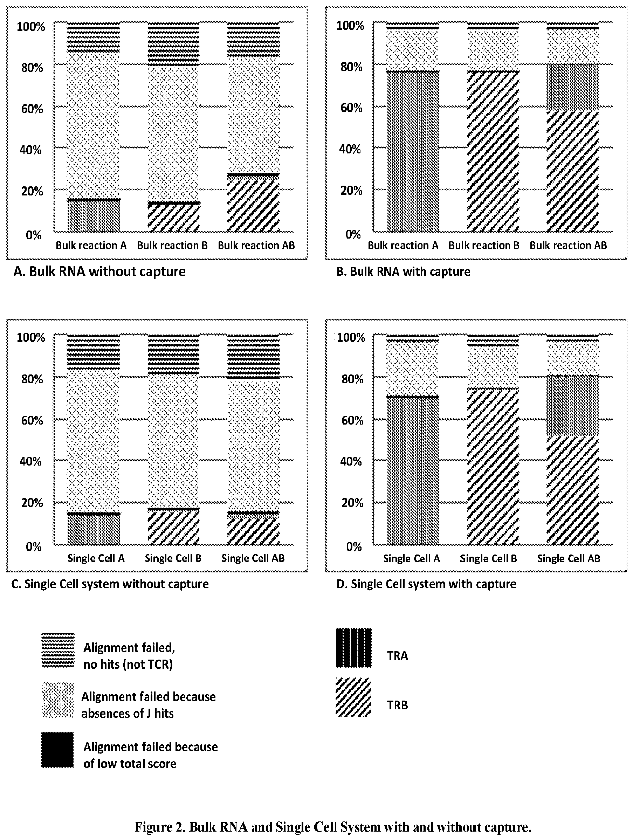

FIG. 2 displays histograms showing next generation sequencing results for TCR alpha chain VDJ, TCR beta chain VDJ, and unrelated to TCR VDJ as a percentage of total reads. (A) Bulk RNA without probe capture. (B) Bulk RNA with probe capture. (C) Single cell without probe capture. (D) Single cell with probe capture.

### Detailed Description

This disclosure generally relates to a method of amplifying TCR alpha chain VDJ and/or TCR beta chain VDJ sequences by reverse transcribing at least one first strand of cDNA from mRNA containing at least one target sequence using an oligo-dT primer, using a multiplex primer mix of primers configured to bind to TCR alpha chain VDJ and/or TCR beta chain VDJ sequences to amplify the cDNA, using probe capture to capture TCR VDJ-specific amplicons and elute non-TCR VDJ-specific amplicons, performing an additional round of PCR to further enrich TCR alpha chain VDJ and TCR beta chain VDJ amplicons, and sequencing the resulting amplicons using next generation sequencing.

As used herein, the term "engineered sequence" refers to a nucleotide sequence. An engineered sequence can be configured for a particular purpose. For example, an engineered sequence can be designed to serve as a universal binding site.

As used herein, the term "oligo-dT" refers to a homopolymer typically consisting exclusively of thymidines. Preferred length of the oligo-dT molecule is 10 to 100 bases, more preferred is a length of 10 to 30 bases, most preferred is a length of 20 bases. The oligo-dT molecule is preferably blocked at its 3'-hydroxyl group by a blocking group to prevent extension by a polymerase reaction. It is known to those of skill in the art that oligo-dT can also be modified as long as the molecule is capable of hybridizing to polyA-RNA. Modifications are, but not limited to, all types of thymidine analogs like 2'-deoxyuridine, 2'-O-methyl-uridine, LNA (locked nucleic acid) thymidine or uridine derivatives, PNA (peptide nucleic acid) thymine or uracil derivatives, HNA (hexitol nucleic acid) thymidine or uridine derivatives or base modified uracil derivatives like 5-propinyl-uracil, but also modification with labels like fluorescent labels or haptens or modification with nucleotides or nucleotide sequences other than thymidine and uridine.

As used herein, the term "probe bead" means a bead to which one or more primers can be adhered and which is suitable for use with one or more purification methods, such as column purification.

As used herein, the term "subject" means a human or other animal. In some embodiments, the subject has been immunized or is suffering from an infection, cancer, an autoimmune condition, or any other diseases. For example, the subject can be a human diagnosed with a disease, exhibiting symptoms of a disease, not diagnosed with a disease, or not exhibiting symptoms of a disease.

As used herein, the term "target sequence" means a nucleic acid sequence to be detected and which anneals with a probe or primer under hybridization, annealing or amplification conditions.

As used herein, the term "TCR alpha chain" and "TCR beta chain" refer to the alpha and beta chains that comprise the TCR and which assemble to form a heterodimer and associate with the CD3-transducing subunits to form the T-cell receptor complex present on the T cell surface. Each alpha and beta chain of the TCR consists of an immunoglobulin-like N-terminal variable (V) and constant (C) region, a hydrophobic transmembrane domain, and a short cytoplasmic region. As for immunoglobulin molecules, the variable region of the alpha and beta chains are generated by V(D)J recombination, creating a large diversity of antigen specificities within the population of T cells.

In certain embodiments, the method of the invention comprises the steps of: reverse transcribing at least one first strand of cDNA from mRNA, wherein the mRNA comprises at least one TCR alpha chain VDJ or TCR beta chain VDJ sequence, using an oligo-dT primer wherein the oligo-dT primer comprises an engineered sequence 5' of the oligo-dT portion. The engineered sequence 5' of the oligo-dT portion provides the benefit of simplifying downstream amplification. The oligo-dT primer may also comprise a molecular barcode as a unique identifier. For example, a molecular barcode can comprise a nucleotide sequence comprising from about 4 to 15 randomly-generated nucleotides. Molecular barcodes can be used as disclosed in US20150132754 (Wang, et al.) and US20120171725 (Han). The mRNA can be isolated from any source comprising one or more T cells. For example, the mRNA can be isolated from peripheral blood or other biological samples from a subject. Methods of extracting mRNA are known to those of skill in the art.

The first strand cDNA produced after reverse transcription with the oligo-dT primer is then amplified using a targeted multiplex mix of primers covering either TCR alpha chain VDJ only or TCR beta chain VDJ only, or both TCR alpha chain and beta chain VDJ. The VDJ-primer mixes may also contain an engineered sequence on the 5' end to provide a universal binding site for downstream PCR. The multiplex PCR mix comprises an optimized primer set of VDJ sequences of interest and a reverse primer for the engineered sequence located on the 5' end of the oligo-dT primer during reverse transcription (Figure 1). In accordance with the invention two or more primers of the multiplex primer mix are an asymmetric balance of primers configured to favor amplification of the sense strand VDJ sequences.

In certain embodiments of the method of the invention, the PCR product of the first amplification is cleaned by either SPRI bead selection or another standard PCR clean-up method, and the PCR is repeated to increase the template amount. The applicants discovered that even if the library was sequenced after the completion of the one or more asymmetric PCR steps, the alpha chain VDJ sequences were still very infrequent in the sequencing data with respect to the beta chain sequences.

Next, the entire PCR product is selected via probe capture. Briefly, probe beads are generated using targeted probe(s) in J or C genes of the TCR alpha chain and the TCR beta chain, for example constant region capture primers specific for the TCR alpha chain constant gene region and the TCR beta chain constant gene region. These capture sequences can be for one specific location or multiple locations within the junction or constant gene for their respective chains. The location of the junction or constant probe primer is also an important consideration as some positions are better than others for VDJ sequence recovery. After the hybridization step, unbound species are washed away - for example by using column purification methods known to those of skill in the art - and the captured sequences are eluted from the beads. Another round of PCR is then performed to both enrich the template again and assign additional indices for sequencing. The library is then sequenced using next generation sequencing, and the data are evaluated for frequency of TCR alpha chain and beta chain VDJ sequences.

During the development of the presently disclosed methods to amplify the VDJ-rearrangements using a multiplex mix of primers covering the TCR beta chain and the TCR alpha chain (a targeted-sequencing approach), the applicants discovered that the VDJ information for the alpha chain was consistently very difficult to capture from an oligo-dT reverse transcribed cDNA, regardless of whether the system was a single cell capture system or a bulk RNA reverse-transcribed by an oligo-dT primer. Since this information forms one of the chains of the receptor pair, it is critical that this information also be recovered.

An advantage of the disclosed methods is to consistently recover both the TCR alpha and beta chain VDJ-rearrangement information using a targeted-sequencing approach coupled to probe-capture. These methods rescue the sequence of the TCR alpha chain VDJ, while also increasing the number of VDJ-specific sequencing reads. As a result, the presently disclosed methods result in a much higher capture of alpha chain sequences relative to conventional methods, thereby increasing the discovery of the alpha chain immune repertoire and the overall receptor pairing success rate. The methods also enable an increased discovery of TCR beta chain sequences. Further, the reduction in the number of off-target sequencing reads offered by the disclosed methods results in overall reduced experiment associated costs, because less sequencing depth is required to maximize the clonotype discovery for each chain. As a result, both immune repertoire discovery and single cell pairing from an oligo-dT reverse transcribed template is improved by the described methods.

### Experiments demonstrating the need for probe-capture

The TCR alpha chain VDJ, the TCR beta chain VDJ, and both together were amplified on reverse transcribed cDNA on bulk RNA. Analysis of the NGS data revealed approximately 90-94% of the data was unrelated to the TCR VDJ sequences, approximately 8% of TCR beta chain sequences and approximately 6% of TCR alpha chain sequences. When the TCR alpha and beta chain sequences were co-amplified, approximately 92% of the data were unrelated to the VDJ sequences, with 5.8% TCR beta chain sequences, and 1.9% TCR alpha chain sequences.

When this experiment was repeated on reverse-transcribed RNA from a single cell system, a similar result was achieved with 89-95% of the data unrelated to the TCR VDJ sequences, 11% of TCR beta chain sequences and 5% of TCR alpha chain sequences. All data unrelated to the VDJ information was considered "trash" since applicants expected, in a targeted sequencing assay, for more than 98% of the data to contain rearranged VDJ sequences. This is the case when a constant region gene primer and a V-primer mix is used during the amplification. This "trash" data results in wasted sequencing reads and dropout of VDJ-rearrangements of interest, thus increasing experiment costs and reducing the VDJ-paired receptor success rates.

### Modifying the primer system and processing in attempt to reduce sequencing trash

After analysis of the NGS data, applicants discovered that much of the trash was a result of one primer, V-alpha 4. Therefore, applicants repeated the experiment using the original primer mix and a primer mix in which V-alpha 4 primer was removed. Applicants also tested multiple ways to wash the beads to help remove any unwanted "trash". Again, analysis of the NGS data revealed only a slight 2% improvement of TCR alpha chain discovery with 92% of the data unrelated to the TCR VDJ sequences (trash) and 8% of TCR alpha chain sequences. Applicants then attempted a different primer set where the V-primers were in a completely different position (closer to the 5' end of the RNA). Again, the same issue persisted regardless of the primer position. Applicants also attempted a method in which the long read primer set was utilized in the first round of PCR, followed by additional PCR with the short read V-primer mix. The idea here being that using two primer sets specific for the V-alpha region would cumulatively result in an increase of V-alpha sequences. This adjustment did not improve the results either.

The off-target sequencing results were re-analyzed for the percentages of various components to see if certain V-alpha sequences could be modified or removed to increase the percentage of on-target V-alpha results. Using all of the information related to the off-target amplicons, the entire primer system was redesigned to avoid off-target results. The analysis on the applicant's bulk RNA oligo-dT system was repeated. Analysis of the NGS data reveals 90% of the data were unrelated to the TCR VDJ sequences (trash) and 6-7% of TCR alpha chain sequences. Thus, even redesigning the primers did not solve the issue of off-target amplification when amplifying the TCR alpha chain VDJ specifically, demonstrating that the TCR alpha chain locus is difficult to amplify with gene-specific primers from cDNA generated with an oligo-dT primer. This difficulty persisted even after modifying the PCR strategy, modifying various PCR clean-up steps, and redesigning the gene-specific primers used for amplification.

### Probe-capture on PCR products

To ascertain whether the issues with the TCR alpha chain were due to inefficient reverse transcription of the TCR alpha chain, applicants tested the first strand cDNA by amplifying with a V- and C- specific primer mix to make sure that the oligo-dT conversion of TCR alpha strand RNA was occurring and to assess the number of TCR alpha chain VDJ sequences that were actually generated as first strand cDNA products. For the same library without performing this enrichment step, applicants discovered approximately 300 clonotypes. However, after enrichment applicants discovered approximately 1,600 clonotypes. These data demonstrate that the TCR alpha chain first strand cDNA is present. Thus, the dropout in TCR alpha chain is happening at a later stage of PCR (not during RT) due to competition from off-target species in the oligo-dT mix.

For Bulk RNA, NGS data reveals by using no probe pull down for TCR alpha chain only, 85% of the data were unrelated to the TCR VDJ (trash). TCR beta chain only reveals 87% of the data were unrelated to the TCR VDJ. When co-amplifying both TCR alpha and beta chain sequences using no probe to capture, 73% of the data were unrelated to the TCR VDJ sequences, 24% of TCR beta chain sequences and 2% of TCR alpha chain sequences (Figure 2-A). Applicants used the probe-capture system after asymmetrical enrichment of second strand cDNA products. The increase in usable TCR alpha and beta chain data with respect to non-probe capture is 7-fold. Using the probe-capture method for TCR alpha and beta chains separately, 23% of data were unrelated to the TCR VDJ sequences. When co-amplifying TCR alpha and beta chain sequences together and capturing with probe, 20% of the data were unrelated to the TCR VDJ sequences, 57% of TCR beta chain sequences and 22% of TCR alpha chain sequences (Figure 2-B).

When this was repeated in the context of a single cell system and the absence of probe-capture with TCR alpha chain, 85% of the data were unrelated to the TCR VDJ sequences (trash). TCR beta chain only reveals 83% of the data were unrelated to the TCR VDJ sequences. When co-amplifying both TCR alpha and beta chain sequences using the non-probe capture system, 85% of the data were unrelated to the TCR VDJ sequences, 11% of TCR beta chain sequences and 3% of TCR alpha chain sequences (Figure 2-C). Applicants used the probe-capture system after asymmetrical enrichment of second strand cDNA products. The increase in usable TCR alpha and beta chain data with respect to non-probe capture is 7-fold. Using the probe-capture method for TCR alpha and beta chain sequences separately, 25% of TCR alpha chain data were unrelated to the TCR VDJ sequences and 29% of TCR beta chain data were unrelated. When co-amplifying TCR alpha and beta chain sequences together and capturing with probe, 19% of the data were unrelated to the TCR VDJ sequences, 51% of TCR beta chain sequences and 28% of TCR alpha chain sequences (Figure 2-D).

The systems, methodologies and the various embodiments thereof described herein are exemplary.

## Claims

1. A method comprising the steps of:
reverse transcribing at least one first strand of cDNA from mRNA using an oligo-dT primer, wherein the mRNA comprises at least one target sequence to produce a first strand cDNA and wherein the oligo-dT primer comprises an engineered sequence on the 5' end;
producing a first set of amplicons by amplifying the first strand cDNA using a multiplex primer mix, wherein the multiplex primer mix comprises two or more primers configured to bind two or more sequences selected from the group consisting of: TCR alpha chain VDJ and TCR beta chain VDJ sequences, and a reverse primer configured to bind to the engineered sequence on the 5' end of the oligo-dT primer wherein the two or more primers of the multiplex primer mix are an asymmetric balance of primers configured to favor amplification of the sense strand VDJ sequences;
using probe beads to capture amplicons comprising TCR alpha chain VDJ sequences and TCR beta chain VDJ sequences from the first set of amplicons, wherein the probe beads comprise targeted probe(s) in J or C genes of the TCR alpha chain and the TCR beta chain;
washing the sample to remove uncaptured amplicons from the first set of amplicons;
eluting captured amplicons from the probe beads to produce a pool of eluted amplicons; and
amplifying the eluted amplicons using PCR to produce a second set of amplicons.

2. The method of claim 1, further comprising the step of, after amplifying the eluted amplicons, sequencing the second set of amplicons using next generation sequencing.

3. The method of claim 2, further comprising the step of, after sequencing the second set of amplicons using next generation sequencing, evaluating the sequences to determine the frequency of TCR alpha chain VDJ sequences and TCR beta chain VDJ sequences.

4. The method of claim 1, wherein the probe bead primers are configured to bind to at least one location within the TCR alpha chain constant gene region and at least one location within the TCR beta chain constant gene region.

5. The method of claim 1, wherein the probe bead primers are configured to bind to at least one location within the TCR alpha chain constant gene region.

6. The method of claim 1, wherein the probe bead primers are configured to bind to at least one location within the TCR beta chain constant gene region.

7. The method of claim 1, wherein the oligo-dT primer comprises a molecular barcode.

8. The method of claim 1, wherein the multiplex primer mix comprises an engineered sequence on the 5' end of the primers and which is configured as a universal binding site.

9. The method of claim 1, wherein, prior to using probe beads, the first set of amplicons is cleaned by SPRI bead selection and an additional round of PCR is performed.

## Patentansprüche

1. Verfahren, umfassend folgende Schritte:
Rücktranskribieren von mindestens einem ersten Strang von cDNA aus mRNA unter Verwendung eines Oligo-dT-Primers, wobei die mRNA mindestens eine Zielsequenz umfasst, um eine cDNA des ersten Strangs zu erzeugen, und wobei der Oligo-dT-Primer am 5'-Ende eine engineerte Sequenz umfasst;
Erzeugen eines ersten Satzes von Amplicons durch Amplifizieren der cDNA des ersten Strangs unter Verwendung eines Multiplex-Primer-Mix, wobei der Multiplex-Primer-Mix zwei oder mehr Primer umfasst, die dazu ausgebildet sind, an zwei oder mehr Sequenzen zu binden, ausgewählt aus der Gruppe bestehend aus: TCR-Alpha-Kette-VDJ- und TCR-Beta-Kette-VDJ-Sequenzen, und eines Reverse-Primer, der dazu ausgebildet ist, an die engineerte Sequenz am 5'-Ende des Oligo-dT-Primers zu binden, wobei die zwei oder mehr Primer des Multiplex-Primer-Mix eine asymmetrische Balance von Primern sind, die dazu ausgebildet sind, die Amplifikation der Sense-Strang-VDJ-Sequenzen zu begünstigen;
Verwenden von Sonden-Beads zum Erfassen von Amplicons, umfassend TCR-Alpha-Kette-VDJ-Sequenzen und TCR-Beta-Kette-VDJ-Sequenzen, aus dem ersten Satz von Amplicons, wobei die Sonden-Beads zielgerichtete Sonde(n) in J- oder C-Genen der TCR-Alpha-Kette und der TCR-Beta-Kette umfassen;
Waschen der Probe, um nicht erfasste Amplicons aus dem ersten Satz von Amplicons zu entfernen;
Eluieren erfasster Amplicons aus den Sonden-Beads, um einen Pool von eluierten Amplicons zu erzeugen; und
Amplifizieren der eluierten Amplicons unter Verwendung von PCR, um einen zweiten Satz von Amplicons zu erzeugen.

2. Verfahren nach Anspruch 1, das weiter den Schritt umfasst, nach dem Amplifizieren der eluierten Amplicons den zweiten Satz von Amplicons unter Verwendung von Sequenzierung der nächsten Generation zu sequenzieren.

3. Verfahren nach Anspruch 2, das weiter den Schritt umfasst, nach dem Sequenzieren des zweiten Satzes von Amplicons unter Verwendung von Sequenzierung der nächsten Generation die Sequenzen auszuwerten, um die Frequenz von TCR-Alpha-Kette-VDJ-Sequenzen und TCR-Beta-Kette-VDJ-Sequenzen zu bestimmen.

4. Verfahren nach Anspruch 1, wobei die Primer der Sonden-Beads ausgebildet sind, an mindestens eine Stelle innerhalb der konstanten Genregion der TCR-Alpha-Kette und an mindestens eine Stelle innerhalb der konstanten Genregion der TCR-Beta-Kette zu binden.

5. Verfahren nach Anspruch 1, wobei die Primer der Sonden-Beads ausgebildet sind, an mindestens eine Stelle innerhalb der konstanten Genregion der TCR-Alpha-Kette zu binden.

6. Verfahren nach Anspruch 1, wobei die Primer der Sonden-Beads ausgebildet sind, an mindestens eine Stelle innerhalb der konstanten Genregion der TCR-Beta-Kette zu binden.

7. Verfahren nach Anspruch 1, wobei der Oligo-dT-Primer einen molekularen Barcode umfasst.

8. Verfahren nach Anspruch 1, wobei der Multiplex-Primer-Mix am 5'-Ende der Primer eine engineerte Sequenz umfasst und die als universelle Bindungsstelle ausgebildet ist.

9. Verfahren nach Anspruch 1, wobei vor dem Verwenden von Sonden-Beads der erste Satz von Amplicons durch SPRI-Bead-Selektion gereinigt wird und eine zusätzliche Runde von PCR durchgeführt wird.

## Revendications

1. Procédé comprenant les étapes suivantes :
transcrire de manière inverse au moins un premier brin d'ADNc à partir d'ARNm en utilisant une amorce oligo-dT, dans lequel l'ARNm comprend au moins une séquence cible pour produire un ADNc de premier brin et dans lequel l'amorce oligo-dT comprend une séquence modifiée à l'extrémité 5' ;
produire un premier ensemble d'amplicons par amplification de l'ADNc de premier brin en utilisant un mélange multiplex d'amorces, dans lequel le mélange multiplex d'amorces comprend deux amorces ou plus conçues pour se lier à deux séquences ou plus sélectionnées dans le groupe consistant en :
des séquences VDJ de chaîne alpha du TCR et de chaîne bêta du TCR, et une amorce inverse conçue pour se lier à la séquence modifiée à l'extrémité 5' de l'amorce oligo-dT, dans lequel les deux amorces ou plus du mélange multiplex d'amorces sont un équilibre asymétrique d'amorces conçues pour favoriser l'amplification des séquences VDJ du brin sens ;
utiliser des billes porteuses de sondes pour capturer des amplicons comprenant des séquences VDJ de chaîne alpha du TCR et des séquences VDJ de chaîne bêta du TCR à partir du premier ensemble d'amplicons, dans lequel les billes porteuses de sondes comprennent une ou plusieurs sondes ciblées dans des gènes J ou C de la chaîne alpha du TCR et de la chaîne bêta du TCR ;
laver l'échantillon pour éliminer des amplicons non-capturés du premier ensemble d'amplicons ;
éluer des amplicons capturés à partir des billes porteuses de sondes pour produire un pool d'amplicons élués ; et
amplifier les amplicons élués en utilisant une PCR pour produire un deuxième ensemble d'amplicons.

2. Procédé de la revendication 1, comprenant en outre l'étape consistant à, après amplification des amplicons élués, séquencer le deuxième ensemble d'amplicons en utilisant un séquençage de nouvelle génération.

3. Procédé de la revendication 2, comprenant en outre l'étape consistant à, après séquençage du deuxième ensemble d'amplicons en utilisant un séquençage de nouvelle génération, évaluer les séquences pour déterminer la fréquence des séquences VDJ de chaîne alpha du TCR et des séquences VDJ de chaîne bêta du TCR.

4. Procédé de la revendication 1, dans lequel les amorces des billes porteuses de sondes sont conçues pour se lier à au moins un emplacement dans la région du gène constant de la chaîne alpha du TCR et à au moins un emplacement dans la région du gène constant de la chaîne bêta du TCR.

5. Procédé de la revendication 1, dans lequel les amorces des billes porteuses de sondes sont conçues pour se lier à au moins un emplacement dans la région du gène constant de la chaîne alpha du TCR.

6. Procédé de la revendication 1, dans lequel les amorces des billes porteuses de sondes sont conçues pour se lier à au moins un emplacement dans la région du gène constant de la chaîne bêta du TCR.

7. Procédé de la revendication 1, dans lequel l'amorce oligo-dT comprend un code-barres moléculaire.

8. Procédé de la revendication 1, dans lequel le mélange multiplex d'amorces comprend une séquence modifiée à l'extrémité 5' des amorces, et qui est conçue comme un site de liaison universel.

9. Procédé de la revendication 1, dans lequel, avant d'utiliser des billes porteuses de sondes, le premier ensemble d'amplicons est nettoyé par sélection sur billes SPRI et un cycle supplémentaire de PCR est effectué.
